# DEMANDE DE BREVET EUROPEEN

(11) **EP 0 914 838 A1**
(43) Date de publication de la demande: **12.05.1999**
(21) Numéro de dépôt: 98402711.0
(22) Date de dépôt: 30.10.1998
(51) Int. Cl.: A61M 25/00, A61B 1/005

(54) **Poignée pour catheter**

(30) Priorité: 30.10.1997 FR 9713636
(71) Demandeur: MEDICORP S.A., 54600 Villers-lès-Nancy (FR)
(72) Inventeur: Amor, Max, 54000 Nancy (FR)
(74) Mandataire: Laget, Jean-Loup

(57) **Abrégé**

Poignée pour cathéter, comportant un système de repérage par index lisibles dans une fenêtre.

La fenêtre 11 est disposée sur la poignée 1, et les index sont portés par une molette 10 pivotant sur un axe perpendiculaire à l'axe du cathéter.

## Description

L'invention concerne une poignée pour cathéter, en particulier pour cathéter d'électrophysiologie.

Un cathéter d'électrophysiologie comporte usuellement un corps de cathéter portant à son extrémité proximale un raccord et à son extrémité distale une partie souple, sur laquelle sont disposées plusieurs électrodes, par exemple annulaires. Ces électrodes sont prévues pour faire des mesures de potentiel, par exemple, en étant appliquées contre la paroi intérieure du coeur ou d'un vaisseau. Elles peuvent aussi être utilisées pour la destruction de certains tissus par électro-ablation ou radio-fréquence.

Le raccord du cathéter comporte à sa partie opposée au corps du cathéter, un connecteur électrique assurant la liaison entre d'une part les conducteurs disposés dans le cathéter et aboutissant aux électrodes, et d'autre part les conducteurs extérieurs aboutissant aux appareils de mesure.

Pour indiquer la position de l'extrémité distale du cathéter, le document US-5.489.275 prévoit de disposer sur le raccord du cathéter une bague cylindrique, coaxiale au cathéter, et munie d'une fenêtre. Lorsque la bague est déplacée en rotation, la fenêtre se déplace devant des index qui correspondent chacun à une position identifiée de l'extrémité distale du cathéter. Ainsi, lorsque le cathéter a été mis dans une position identifiée, l'opérateur fait pivoter la bague jusqu'à ce que la fenêtre soit devant l'index correspondant à la position identifiée. L'inconvénient de cette disposition coaxiale d'une bague cylindrique est dû à la position variable de la fenêtre, qui n'est pas toujours sur le côté du raccord du cathéter le plus visible par l'opérateur.

Un but de l'invention est de proposer une poignée de cathéter pour faciliter la visibilité d'un index de repérage sur sa face visible par l'opérateur.

Dans les cathéters orientables, un dispositif de commande permet d'assurer l'orientation de l'extrémité distale du cathéter. Ce dispositif porte une indication graduée, plus ou moins précise, liée à la position du dispositif de commande.

Un autre but de l'invention est de proposer une poignée pour cathéter orientable, qui permette l'affichage d'un index de repérage de la position de l'extrémité distale du cathéter, par exemple sa courbure ou sa position angulaire.

L'invention a pour objet une poignée pour cathéter, comportant un système de repérage par index lisibles dans une fenêtre, caractérisée en ce que la fenêtre est disposée sur la poignée, et les index sont portés par une molette pivotant sur un axe perpendiculaire à l'axe du cathéter. Ainsi, la ou les fenêtres sont toujours situées aux mêmes endroits par rapport à la poignée, ce qui facilite leur visibilité par l'utilisateur.

Selon d'autres caractéristiques :
- la poignée présente un évidement dans lequel est logée la molette, les index portés par la molette défilant sous la fenêtre ;
- la poignée présente une deuxième fenêtre et les index sont portés par les deux faces de la molette, pour assurer la réversibilité de la poignée ;
- la molette est indépendante de la commande d'orientation du cathéter et le système de repérage donne une information de position de l'extrémité distale du cathéter ;
- une liaison mécanique est prévue entre la molette et la commande d'orientation du cathéter, et le système de repérage donne une information angulaire de l'extrémité distale de la partie orientable du cathéter par rapport à l'extrémité distale du corps du cathéter ou une indication sur la courbure de l'extrémité distale du cathéter.

D'autres caractéristiques ressortent de la description qui suit faite avec référence aux dessins annexés dans lesquels :
- la Figure 1 est une vue de dessus d'un premier mode de réalisation d'une poignée pour cathéter, selon l'invention ;
- la Figure 2 est une vue de côté de la poignée pour cathéter de la Figure 1 ;
- la Figure 3 est une vue en perspective d'un support de molette pour poignée pour cathéter selon la Figure 1 ;
- la Figure 4 est une vue en plan d'une molette pour poignée pour cathéter selon la Figure 1 ;
- la Figure 5 est une vue de dessus d'un deuxième mode de réalisation d'une poignée pour cathéter, selon l'invention ;
- la Figure 6 est une vue de côté de la poignée pour cathéter selon la Figure 5.

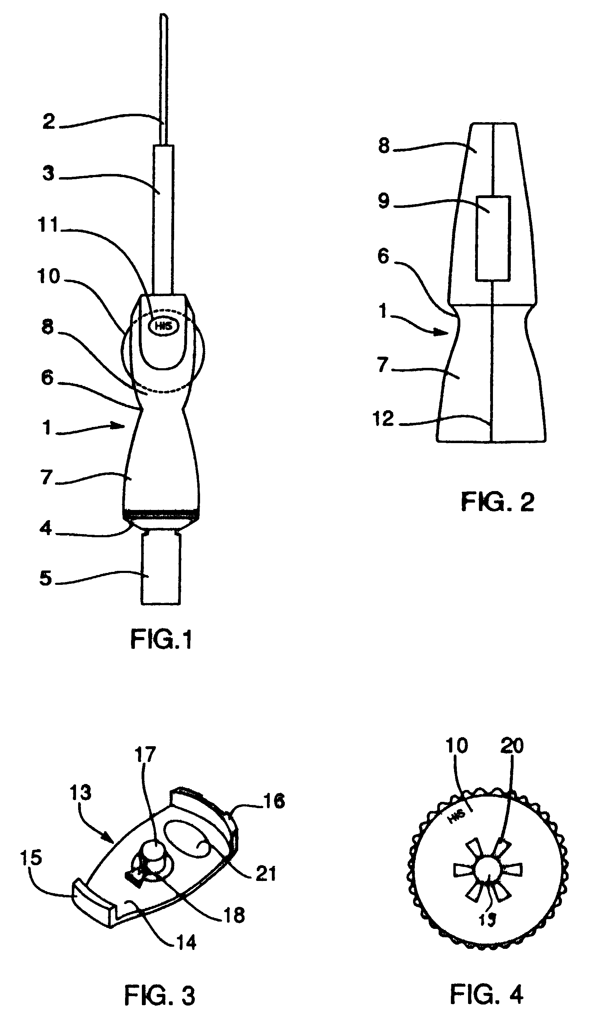

Sur la Figure 1, la poignée 1 pour cathéter se trouve à l'extrémité proximale d'un cathéter 2. Elle porte à l'avant un manchon 3 de protection du cathéter au voisinage de la poignée 1, et à l'arrière un connecteur électrique 4 pour assurer la liaison entre d'une part les conducteurs intérieurs au cathéter et venant des électrodes disposées à l'extrémité distale du cathéter, et d'autre part les conducteurs extérieurs au cathéter, logés dans une gaine 5, et reliés aux appareils de mesure et/ou d'enregistrement d'un électrocardiogramme par exemple.

La poignée 1 présente une partie centrale étroite 6, à l'arrière une partie sensiblement tronconique 7, et à l'avant une partie galbée 8 aplatie. Cette partie galbée 8 présente un évidement central 9, visible sur la Figure 2, dans lequel sont logés une molette 10 (Figure 4) et son support 13 (Figure 3). La molette 10 est disposée dans l'évidement central 9, et son diamètre est supérieur à la largeur de la partie avant galbée 8 de la poignée 1, de sorte que la molette 10 dépasse latéralement de part et d'autre de la partie avant 8 de la poignée. La partie avant 8 de la poignée 1 présente une fenêtre 11, sensiblement dans l'axe de la poignée, au dessus de la molette 10, et vers l'avant de la poignée 1. Cette fenêtre 11 permet de voir les index inscrits sur la molette 10, et qui défilent sous la fenêtre 11 lorsque la molette 10 est actionnée en rotation par la main de l'opérateur agissant sur les parties de la molette 10 qui dépassent latéralement de part et d'autre de la partie avant 8 de la poignée 1.

Sur la Figure 2, la poignée 1 apparaît constituée de deux moitiés séparées par un plan médian 12. Les mêmes références désignent les mêmes parties que sur la Figure 1.

Sur la Figure 3, le support 13 de la molette 10 comporte un fond 14 et deux bordures en arc de cercle 15 et 16. Au centre du fond 14 est disposé un pivot 17, et sur le fond, à côté du pivot 17, est prévu un ergot 18. Le support 13 de molette est destiné à s'encliqueter dans la partie avant 8 de la poignée 1 et à recevoir la molette 10, centrée sur le pivot 17 et pivotant à l'intérieur des bordures 15, 16 en arc de cercle.

Sur la Figure 4, la molette 10 se présente comme une roue munie de reliefs sur sa périphérie. Elle comporte un orifice central 19 destiné à recevoir le pivot 17 du support 13. Autour de cet orifice central 19, et rayonnant vers l'extérieur, sont disposées des encoches 20 destinées à coopérer avec l'ergot 18 pour immobiliser la molette 10 dans des positions correspondant à la présence des index sous la fenêtre 11 de la poignée 1. Sur la surface de la molette 10, en regard des encoches 20, sont disposés des index d'identification de la position de l'extrémité distale du cathéter, par exemple. Un index est par exemple HIS pour indiquer la présence de l'extrémité distale du cathéter dans le faisceau de His. Les autres index correspondent par exemple à la présence de l'extrémité distale du cathéter dans l'oreillette droite, dans le ventricule droit, et ainsi de suite.

Pour assurer la réversibilité de la poignée 1, chacune des deux moitiés de la poignée (Figure 2) peut présenter une fenêtre telle que 11. Le support 13 de molette peut alors présenter une fenêtre 21 correspondant à la fenêtre 11 et la molette 10 peut porter des index sur ses deux faces.

Dans ce premier mode de réalisation, la molette 10, dont l'axe de rotation est perpendiculaire à l'axe du cathéter dans la poignée 1, est mise en position par l'opérateur pour garder une information. Cette information correspond par exemple à la position de l'extrémité du cathéter. Cette information est utile à l'opérateur, notamment lorsqu'il y a simultanément plusieurs cathéters dans un coeur.

Dans ce premier mode de réalisation, la molette est indépendante de la commande d'orientation de l'extrémité distale du cathéter. Le système de repérage de position constitué par la molette 10 et ses index sert d'aide-mémoire à l'opérateur.

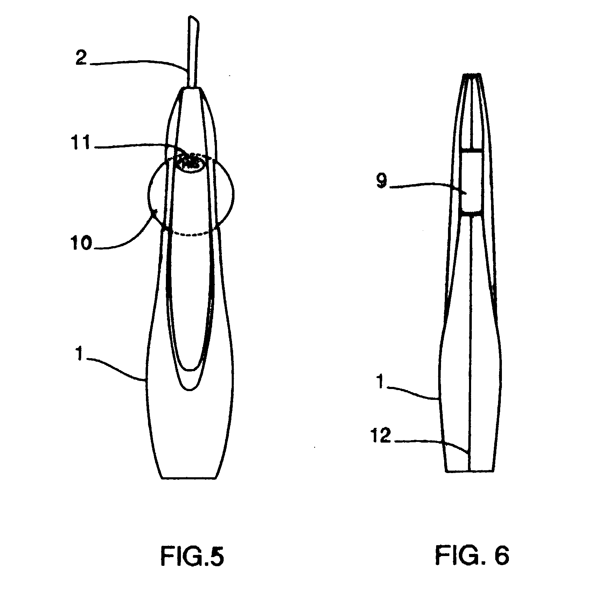

Sur les Figures 5 et 6, la poignée 1 du cathéter 2 a une forme plus allongée que sur la Figure 1. Elle est cependant réalisée en deux moitiés séparées par un plan médian 12, et elle présente à sa partie avant un évidement central 9, et une fenêtre 11 pour l'observation des index marqués sur la molette 10.

Dans le deuxième mode de réalisation correspondant aux Figures 5 et 6, le cathéter présente un corps de cathéter ou partie non orientable du cathéter, et à l'extrémité distale de ce corps, une partie souple de cathéter, ou partie orientable du cathéter.

Les index portés par la molette 10 sont des indications angulaires correspondant à l'orientation de l'extrémité distale de la partie souple du cathéter par rapport à l'extrémité distale du corps du cathéter ou des indications de la courbure du tube distal ou de la partie distale du cathéter.

Dans ce deuxième mode de réalisation, une liaison mécanique est prévue entre la molette 10 et le moyen de commande de l'orientation de l'extrémité distale de la partie souple du cathéter. De cette manière, l'information fournie par la molette 10 est corrélée à l'orientation de l'extrémité distale de la partie souple du cathéter. Cette orientation est définie comme l'angle formé par la tangente à l'extrémité distale de la partie souple du cathéter et la tangente à l'extrémité distale du corps du cathéter ou comme la courbure de la partie distale du cathéter.

Ainsi, la poignée 1 de cathéter est munie d'un système de repérage de la position de l'extrémité distale du cathéter. Ce système est constitué par une molette 10, logée dans une poignée 1, susceptible de pivoter autour de son axe, et dotée d'index sur l'une au moins de ses faces. Ces index peuvent être des signes, des symboles, des chaînes de caractères compréhensibles par l'opérateur. Ces index sont visibles par au moins une fenêtre de la poignée 1. Lorsqu'il y a deux fenêtres dans la poignée 1, elles permettent d'observer simultanément les deux faces de la molette et d'y voir les mêmes index.

La position angulaire de la molette 10 correspond à une indication utile à l'opérateur. Cette indication peut être l'endroit du coeur ou d'une artère dans lequel est située l'extrémité distale du cathéter. Elle peut être aussi l'angle formé par la tangente à l'extrémité distale du corps de cathéter (ou partie non orientable du cathéter) et la tangente à l'extrémité distale de la partie souple du cathéter (ou partie orientable du cathéter).

## Revendications

1. Poignée pour cathéter, comportant un système de repérage par index lisibles dans une fenêtre, caractérisée en ce que la fenêtre (11) est disposée sur la poignée (1), et les index sont portés par une molette (10) pivotant sur un axe perpendiculaire à l'axe du cathéter.

2. Poignée selon la revendication 1, caractérisée en ce que la poignée (1) présente un évidement (9) dans lequel est logée la molette (10), les index portés par la molette (10) défilant sous la fenêtre (11).

3. Poignée selon la revendication 2, caractérisée en ce que la poignée (1) présente une deuxième fenêtre et les index sont portés par les deux faces de la molette (10), pour assurer la réversibilité de la poignée (1).

4. Poignée selon la revendication 2, caractérisée en ce que la molette (10) est indépendante de la commande d'orientation du cathéter et le système de repérage donne une information de position de l'extrémité distale du cathéter.

5. Poignée selon la revendication 2, caractérisée en ce que une liaison mécanique est prévue entre la molette (10) et la commande d'orientation du cathéter, et le système de repérage donne une information angulaire de l'extrémité distale de la partie orientable du cathéter par rapport à l'extrémité distale du corps du cathéter ou une indication sur la courbure de l'extrémité distale du cathéter.
